Europäisches Patentamt

European Patent Office

Office européen des brevets

(11) Publication number: **0 169 377 B1**

## EUROPEAN PATENT SPECIFICATION

(45) Date of publication of patent specification: **05.02.92**

(51) Int. Cl.⁵: **C12N 15/77**, C12N 15/54, C12N 15/65

(21) Application number: **85107645.5**

(22) Date of filing: **20.06.85**

(54) Plasmid and bacteria containing the same.

(30) Priority: **22.06.84 JP 128830/84**
**27.12.84 JP 281341/84**

(43) Date of publication of application:
**29.01.86 Bulletin 86/05**

(45) Publication of the grant of the patent:
**05.02.92 Bulletin 92/06**

(84) Designated Contracting States:
**DE FR GB IT**

(56) References cited:
**EP-A- 0 082 485**
**GB-A- 2 076 853**

**JOURNAL OF BIOCHEMISTRY, vol. 91, no. 4, April 1982, pages 1205-1212, Tokyo, JP; M. IWAKURA et al.: "Cloning of dihydrofolate reductase gene of Escherichia coli K12"**

(73) Proprietor: **AJINOMOTO CO., INC.**
**5-8, Kyobashi 1-chome, Chuo-ku**
**Tokyo 104(JP)**

(72) Inventor: **Takagi, Hiroshi**
**No. 1155-2, Nakamaruko Nakahara-ku**
**Kawasaki-shi Kanagawa-ken(JP)**
Inventor: **Miwa, Kiyoshi**
**3-304, Sagamidai-Jutaku No. 531, Iwase**
**Matsudo-shi Chiba-ken(JP)**
Inventor: **Nakamori, Shigeru**
**No. 2153-187, Kamariya-cho**
**Yokohama-shi Kanagawa-ken(JP)**
Inventor: **Sano, Konosuke**
**No. 1-35-9, Uehara**
**Shibuya-ku Tokyo(JP)**

(74) Representative: **Strehl, Schübel-Hopf, Groening**
**Maximilianstrasse 54 Postfach 22 14 55**
**W-8000 München 22(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 169 377 B1

MOLECULAR AND CELLULAR BIOLOGY, vol. 4, no. 3, March 1984, pages 407-414, American Society for Microbiology; A. MIYAJIMA et al.: "Expression of plasmid R388-encoded type II dihydrofolate reductase as a dominant selective marker in Saccharomyces cerevisiae"

GENE, vol. 29, no. 1, July 1984, pages 135-143, Elsevier Science Publishers; T.T. MYODA et al.: "Cloning and mapping of the dihydrofolate reductase gene of Bacillus subtilis"

AGRICULTURAL AND BIOLOGICAL CHEMISTRY, vol. 50, no. 10, October 1986, pages 2597-2603, Tokyo, JP; H. TAKAGI et al.: "Versatile cloning vectors constructed with genes indigenous to a glutamic acid-producer, Brevibacterium lactofermentum"

**Description**

BACKGROUND OF THE INVENTION

Field of the Invention

The present invention relates to plasmids and bacteria containing them and more particularly to plasmid vectors capable of replicating in Coryneform bacterial cells and to Coryneform bacteria containing them.

Discussion of the Background

Coryneform bacteria include bacteria, such as Brevibacterium flavum, that produce amino acids such as glutamic acid in large quantities and are an extremely important group of microorganisms from an industrial viewpoint.

In order to enhance productivity of fermentation products of Coryneform bacteria through recombinant DNA techniques, several plasmid vectors have already been developed utilizing Coryneform bacteria as host cells (see, for example, the First Publication of European Patent Application 9 093 611). These plasmids all were prepared by inserting marker genes for selection of a plasmid into a cryptic plasmid that can replicate in Coryneform bacterial cells. For this reason, foreign genes derived from the genus Escherichia, the genus Bacillus, and the like have been inserted into plasmids of Coryneform bacteria as marker genes. Accordingly, for Coryneform bacteria, DNA alien to the host has been integrated therein. The use of such plasmid vectors corresponds to a recombination of alien or extraneous DNA into the host.

SUMMARY OF THE INVENTION

Therefore, an object of the present invention is to produce recombinant plasmid vectors capable of replication in Coryneform bacterial cells and comprising a first segment comprising a replication site capable of causing replication of said plasmid in a Coryneform bacterial cell and a second segment comprising a Brevibacterium lactofermentum gene capable of conferring trimethoprim resistance and obtainable from any of the plasmids pAJ 228 or pAJ 224 (FERM-BP 786 and 787, respectively). A further object of the present invention is to provide a method of producing a recombinant plasmid capable of transforming a Coryneform bacterium, which comprises ligating (1) a DNA segment containing a replication site capable of causing replication of a plasmid in a Coryneform bacterial cell with (2) a DNA segment comprising a Brevibacterium lactofermentum gene capable of conferring trimethoprim resistance obtainable from any of the plasmids pAJ 228 deposited in strain AJ 12 147 (FERM-P 7673 = FERM-BP 786) or pAJ 224 deposited in strain AJ 12 196 (FERM-P 8015 = FERM-BP 787). A still further object of the present invention is to provide a transformed Coryneform bacterium containing any of the above recombinant plasmids.

In order to solve the foregoing problem, the present inventors have produced plasmids which replicate in Coryneform bacterial cells and possess a gene which, when introduced in the cells, expresses trimethoprim resistance and which is derived from Coryneform bacteria. Trimethoprim is a compound having a structure analogous to (and antagonist activity against) dihydrofolic acid and is an inhibitor of dihydrofolic acid dehydrogenase.

BRIEF DESCRIPTION OF THE DRAWINGS

A more complete appreciation of the invention and many of its attendant advantages will be readily obtained by reference to the following detailed description when considered in connection with the accompanying drawings, wherein:
Figure 1 is a restriction map of plasmid pAJ 228;
Figure 2 is a restriction map of plasmid pAJ 225;
Figure 3 is a restriction map of plasmid pAJ 226;
Figure 4 is a restriction map of plasmid pAJ 224 for expression;
Figure 5 is a restriction map of plasmid pAJ 224;
Figure 6 is a restriction map of plasmid pAJ 224 Cm$^r$; and
Figure 7 is a restriction map of plasmid pAJ 223.

DETAILED DESCRIPTION OF THE INVENTION

In the present invention, marker genes present in the plasmids are derived from Coryneform bacteria and are therefore referred to as "intraneous DNA." The phrase "intraneous DNA" refers to DNA that has been derived from the cell type in which the plasmid vector carrying this DNA insert normally replicates. In other words, the DNA is derived from the host organism into which the recombinant plasmid is to be inserted. Thus, the intraneous DNA as well as the other parts of the recombinant plasmid vector are not foreign to the host as they all originate from the same source.

The gene which expresses trimethoprim-resistance when introduced into Coryneform bacterial cells is obtained as follows. First, a strain having resistance to trimethoprim is selected from Coryneform bacteria. Wild strains of Coryneform bacteria are generally sensitive to trimethoprim. It is thus preferred that a mutant be induced in order to obtain a strain resistant to trimethoprim.

Mutants having resistance to trimethoprim may readily be obtained, e.g. by exposing the parent strain to mutation-inducing agents such as N-methyl-N'-nitro-N-nitrosoguanidine, etc., followed by selecting strains that have acquired trimethoprim-resistance from a culture medium containing trimethoprim. The strains which acquire trimethoprim-resistance may be obtained by adding at least 100 $\mu$g/ml, preferably over 200 $\mu$g/ml, of trimethoprim to the culture medium. A typical medium, for example, is minimum medium, which contains 2 g/dl of glucose, 1 g/dl of ammonium sulfate, 0.25 g/dl of urea, 0.1 g/dl of $KH_2PO_4$, 0.04 g/dl of $MgSO_4.7H_2O$, 2 ppm of ferric ions, 2 ppm of manganese ions, 200 $\mu$g/l of thiamine hydrochloride, 50 $\mu$g/l of biotin, and 1.5 g/dl of agar and has been adjusted to a pH of 7.0. Resistant strains are readily obtained by selecting strains that grow on the medium.

For separating the genes expressing trimethoprim resistance, chromosomal genes are first extracted (for example, a method described in H. Saito and K. Miura, Biochem. Biophys. Acta, 72, 619 (1963) can be used) from strains of Coryneform bacteria having genes expressing trimethoprim-resistance, e.g. from trimethoprim-resistant Brevibacterium lactofermentum AJ 12146, FERM-BP 785. The chromosomal genes are then cleaved with an appropriate restriction enzyme, and are ligated with plasmid vectors capable of replicating in Coryneform bacteria. Using the resulting recombinant DNAs, Coryneform bacteria are transformed, and strains which come to possess trimethoprim-resistance are separated using techniques such as those described above. From the growing strains, genes expressing trimethoprim-resistance can be separated using standard techniques.

Coryneform bacteria used as hosts for transformation in the selection step described above are those sensitive to trimethoprim. Therefore, transformants having genes expressing trimethoprim resistance can be obtained as strains resistant to trimethoprim, as has been described above.

Coryneform bacteria are aerobic, gram-positive rods, non-acid fast and are described in Bergey's Manual of Determinative Bacteriology, 8th Edition, 599 (1974). Among them, bacteria producing L-glumatic acid in large quantities as exemplified below are known and are all deemed to belong to the same genus.

Brevibacterium divaricatum ATCC 14020
Brevibacterium saccarolyticum ATCC 14066
Brevibacterium immariophilum ATCC 14068
Brevibacterium lactofermentum ATCC 13869
Brevibacterium roseum ATCC 13825
Brevibacterium flavum ATCC 13826
Brevibacterium thiogenitalis ATCC 19240
Corynebacterium acetoacidophilum ATCC 13870
Corynebacterium acetoglutamicum ATCC 15806
Corynebacterium callunae ATCC 15991
Corynebacterium glutamicum ATCC 13032, 13060
Corynebacterium lilium ATCC 15990
Corynebacterium melassecola ATCC 17965
Corynebacterium ammoniaphilum ATCC 15354

In addition to the above-mentioned glutamic-acid-producing Coryneform bacteria, Coryneform bacteria include mutants which have lost glutamic acid productivity and other mutants.

To obtain plasmids capable of replicating in Coryneform bacterial cells, the gene expressing trimethoprim-resistance is ligated with a replication region of a plasmid obtained from a Coryneform bacterial cell. As replication region DNAs, all wild-type plasmids capable of replicating in Coryneform bacterial cells or DNA fragments containing the replication region of such plasmids can be employed. In addition, plasmids obtained by ligating other Coryneform bacterial-cell-derived DNAs with these wild-type plasmids or with the DNA fragments containing the replication region can also be used as the replication region DNAs.

As the wild-type plasmids capable of replicating in Coryneform bacterial cells, there are known pAM 330 and pAM 286 described in Published Unexamined Japanese Patent Application 67699/83, pHM 1519 described in Published Unexamined Japanese Patent Application 77895/83, pCG 2 described in Published Unexamined Japanese Patent Application 35197/83, pCG 4 and pCG 11 described in Published Unexamined Japanese Patent Application 183799/83, and pCG 1 described in Published Unexamined Japanese Patent Application 134500/82.

The plasmids having replication region DNAs are typically cleaved with the same restriction enzyme used for cleaving the chromosomal genes in order to produce plasmid terminals capable of ligating with the DNA that encodes trimethoprim-resistance. The chromosomal-DNA cleaved fragments and cleaved plasmid DNAs are subjected to a ligation reaction as they are or, if necessary, after connecting them with an oligonucleotide having complementary base sequences at each of the terminals.

To introduce the thus-obtained recombinant DNA comprising the chromosomal DNA and the plasmid DNA into recipient bacteria of the Coryneform genus, it is possible to use, for example, a process which comprises treating recipient cells with calcium chloride to increase the permeability of DNA as reported for Escherichia coli K-12 (Mandel, M. and Higa, A., J. Mol. Biol., 53, 159 (1970)); a process which comprises introducing recombinant DNA into a propagation stage (so called competent cell) at which time cells can integrate DNA, as reported for Bacillus subtilis (Duncan, D.H., Wilson, G.A. and Young, F.E., Gene, 1, 153 (1977)); and other known methods. Alternatively, as is know with respect to actinomyces and yeasts (Chang, S. and Cohen, S.N., Molec. Gen. Genet., 168, 111 (1979); Bibb, M.J., Ward, J.M. and Hopwood, O.A., Nature, 274, 398 (1978); Hinnen, A., Hicks, J.B. and Fink, G.R., Proc. Natl. Acd. Sci. USA 75, 1929 (1978)), it is also possible to change DNA recipients into protoplasts or spheroplasts capable of easily incorporating plasmid DNA and to introduce the DNA in this fashion.

In the protoplast technique, sufficiently high frequency can be obtained even by the above-described process used for Bacillus subtilis. Of course, a process which comprises allowing DNA to incorporate into protoplasts of the genus Corynebacterium or the genus Brevibacterium described in Published Unexamined Japanese Patent Application 183799/82 in the presence of polyethylene glycol or polyvinyl alcohol and divalent metal ions can also be utilized. Similar results can also be obtained by a process in which the incorporation of DNA is accelerated by the addition of carboxymethyl cellulose, dextran, phycoll, Pluronic F 68 (Serva Co., Ltd.), etc., in place of polyethylene glycol or polyvinyl alcohol.

After transformation, strains having acquired trimethoprim-resistance are separated as the desired transformants. Such transformants contain recombinant DNA having incorporated therein genes expressing trimethoprim resistance. To isolate the recombinant DNA, the transformants are lysed, for example by a treatment with lysozyme and SDS, and then treated with phenol. Two volumes of ethanol are then added thereto to precipitate and recover the recombinant DNA.

The thus-obtained plasmids capable of replicating in Coryneform bacterial cells, which express trimethoprim-resistance when introduced into the cells, contain no heterogenic DNA and are thus particularly suited for inserting genes derived from Coryneform bacteria into a host Coryneform bacteria for expression. As genes to be inserted and expressed, genes participating in biosynthesis of amino acids and the like are deemed to be particularly suitable.

The invention now being generally described, the same will be better understood by reference to certain specific examples which are included herein for purposes of illustration only and are not intended to be limiting of the invention or any embodiment thereof, unless specified.

Example 1

Construction of pAJ 228

(1) Trimethoprim-resistant mutant AJ 121246 (FERM-P 7672 FERM BP-785), mutation-derived from Brevibacterium lactofermentum AJ 12036, was inoculated on 1 liter CMG medium, which contained 1 g/dl of peptone, 1 g/dl of yeast extract, 0.5 g/dl of glucose, and 0.5 g/dl of NaCl and had been adjusted to pH of 7.2. Shake culture was conducted at 30°C for about 3 hours, and cells were harvested at a logarithmic growth phase. After the cells were lysed by lysozyme and SDS, chromosomal DNAs were extracted and purified by the conventional phenol method to finally obtain 3.0 mg of DNAs.

(2) As the vector, pAM 330 was used. Plasmid pAM 330 was prepared as follows. First, Brevibacterium lactofermentum ATCC 13869 containing pAM 330 as plasmids was inoculated on 100 ml of CMG medium. After culturing at 30°C to reach a late logarithmic growth phase, the cells were lysed by lysozyme and SDS. The supernatant was obtained by ultracentrifugation at 30,000 x g for 30 minutes. After treatment with phenol, 2 volumes of ethanol were added to recover DNAs as precipitates. After the DNAs were dissolved in a small quantity of TEN buffer (20 mM tris hydrochloride, 20 mM NaCl, 1 mM EDTA, pH 8.0), the solution was subjected to agarose gel electrophoresis to separate the DNAs. Thereafter, the separated product was taken out to obtain about 15 $\mu$g of pAM 330 plasmid DNAs.

(3) The chromosomal DNAs, 20 ug, obtained in step (1) and 10 $\mu$g of the plasmid DNAs obtained in step (2) were separately treated with restriction endonuclease Mbo I at 37°C for 30 minutes to effect partial cleavage. After heat treatment at 65°C for 10 minutes, both reaction solutions were mixed with each other, and the mixture was subjected to a ligation reaction of DNA strands with $T_4$-phage-derived DNA ligase at 10°C for 24 hours in the presence of ATP and dithiothreitol. After heat treatment at 65°C for 5 minutes, a 2-fold volume of ethanol was added to the reaction solution to precipitate and harvest DNAs after completion of the ligation reaction.

(4) Brevibacterium lactofermentum AJ 12036 (FERM-BP 734) sensitive to trimethoprim was used as the recipient. As the transformation method, a protoplast transformation method was used. First, the cells were cultured in 5 ml of CMG liquid medium to reach an earlier logarithmic growth phase. After adding 0.6 unit/ml of penicillin, shake culture was conducted for a further 1.5 hour. Cells were harvested by centrifugation and washed with 0.5 ml of SMMP medium (pH 6.5) composed of 0.5 M sucrose, 20 mM maleic acid, 20 mM magnesium chloride and 3.5% Pennassay broth (Difco) and then suspended in SMMP medium containing 10 mg/ml of lysozyme. The suspension was treated at 30°C for 20 hours to obtain protoplasts. After centrifuging at 6000 x g for 10 minutes, the protoplasts were washed with SMMP and resuspended in 0.5 ml of SMMP. The thus-obtained protoplasts were mixed with 10 $\mu$g of DNAs prepared in step (3) in the presence of 5 mM EDTA. After polyethylene glycol was added to the mixture to reach a final concentration of 30%, the mixture was allowed to stand at room temperature for 2 minutes to incorporate DNAs into the protoplasts. After the protoplasts were washed with 1 ml of SMMP medium, the protoplasts were resuspended in 1 ml of SMMP, and the suspension was cultured at 30°C for 2 hours for phenotypic expression. The culture solution was spread onto a protoplast regeneration medium of pH 7.0. The regeneration medium contained, per one liter of distilled water, 12 g of tris-(hydroxymethyl)-aminomethane, 0.5 g of KCl, 10 g of glucose, 8.1 g of $MgCl_2.6H_2O$, 2.2 g of $CaCl_2.2H_2O$, 4 g of peptone, 4 g of yeast extract powder, 1 g of Casamino Acid (Difco Co., Ltd.), 0.2 g of $K_2HPO_4$, 135 g of sodium succinate, 8 g of agar, and 25 $\mu$g/ml of trimethoprim (Sigma Co., Ltd.).

After culturing at 30°C for 1 week, about 100 colonies appeared, which were replicated in minimum medium (which contained 2% of glucose, 1% of ammonium sulfate, 0.25% of urea, 0.1% of $KH_2PO_4$, 0.04% of $MgSO_4.7H_2O$, 2 ppm of ferric ions, 2 ppm of manganese ions, 200 $\mu$g/l of thiamine hydrochloride and 50 $\mu$g/l of biotin, pH 7.0, 1.8% of agar, and 50 $\mu$g/ml of trimethoprim) to obtain strains resistant to trimethoprim.

(5) From a strain obtained in this manner, a lysate was prepared by the method described in step (2). When plasmid DNAs were detected by agarose gel electrophoresis, a plasmid obviously larger than vector pAM 330 was present. This strain was named AJ 12147 (FERM-P 7673, FERM-BP 786).

(6) To confirm that a trimethoprim-resistance gene was present in the parent plasmid (PAJ 228) possessed by AJ 12147, Brevibacterium lactofermentum AJ 12036 was again transformed using the plasmid DNA.

Among colonies appearing which had trimethoprim-resistance, 10 cells each were selected and subjected to agarose gel electrophoresis to detect the plasmid DNA. In all of them, plasmids having the same size as that of pAJ 228 were present. It became clear that the gene expressing trimethorim-resistance was present on the recombinant plasmid described above.

Brevobacterium lactofermentum AJ 12146 (FERM-P 7672) was obtained by contacting Brevibacterium lactofermentum AJ 12036 with 1,000 $\mu$g/ml of N-methyl-N'-nitro-N-nitrosoguanidine at 0°C for 20 minutes and separating as cells capable of growing in minimum medium (which contained 2 g/dl of glucose, 1 g/dl of ammonium sulfate, 0.25 g/dl of urea, 0.1 g/dl of $KH_2PO_4$, 0.04 g/dl of $MgSO_4.7H_2O$, 200 $\mu$g/l of thaimine hydrochloride, 50 $\mu$g/l of biotin, 2 ppm of ferric ions, 2 ppm of manganese ions and 1.5 g/dl of agar and had been adjusted to pH of 7.0) containing 100 $\mu$g/ml of added trimethoprim.

AJ 12036 was isolated from the colonies of the wild strain Brevibacterium lactofermentum ATCC 13869 as the strain which spontaneously obtained resistance to streptomycin and spontaneously lost plasmid pAM 330, which was possessed by ATCC 13869.

(7) Properties of pAJ 228 DNA

(a) The molecular weight of pAJ 228 was determined by agarose gel electrophoresis. Agarose gel electrophoresis was performed in accordance using the method of P.A. Sharp et al. (Biochemistry, 12, 3055 (1973)) using 0.8% gel at a constant voltage of 5 V per cm of gel length for 15 hours. The molecular weight was calculated by a comparison of mobility with a molecular weight marker having known molecular weight,λ phage Hind III fragment (BRL Co., Ltd.) after reacting 0.5 unit of restriction enzyme Cla I for cleaving 1 portion of pAJ 228 with 0.5 $\mu$g of pAJ 228 at 37°C for 1 hour. The molecular size was determined to be 7.6 Kb. Further, the number of cleaved segments (cleavage sites) with various restriction enzymes commercially available is shown in Table 1.

Table 1

| Restriction Enzyme | Number of Cleaved Segments |
|---|---|
| Ava I | 5 |
| BamH I | 0 |
| Bcl I | 4 |
| Bgl II | 0 |
| BstE II | 3 |
| Cla I | 1 |
| EcoR I | 0 |
| Hae II | 4 |
| Hind III | 2 |
| Hpa I | 1 |
| Kpn I | 0 |
| Mlu I | 2 |
| Pst I | 0 |
| Pvu II | 0 |
| Sal I | 0 |
| Sca I | 2 |
| Sma I | 1 |
| Sst I | 0 |
| Sst II | 0 |
| Xba I | 1 |
| Xma I | 1 |
| Xor II | 0 |
| Xho I | 1 |

(b) Preparation of a restriction map of pAJ 228 DNA

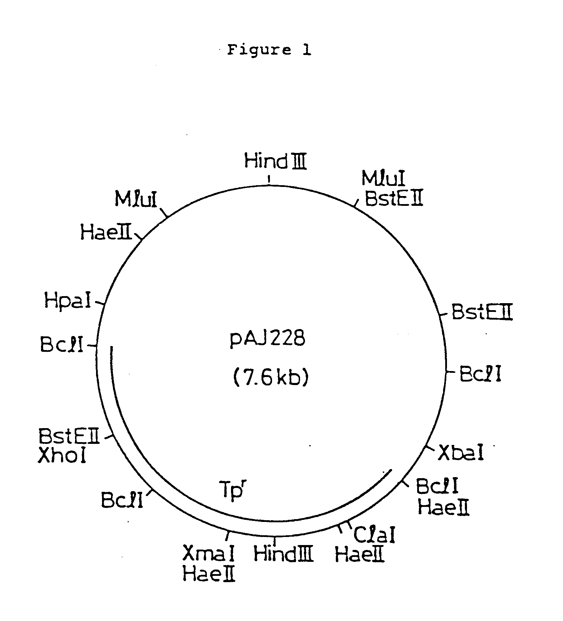

Commercially available enzymes from BRL were used as restriction enzymes and cleavage of pAJ 288 DNA with each restriction enzyme was carried out using at least a 3-fold excess of enzyme under given conditions with respect to each enzyme. When plasmid DNA was cleaved with one or more restriction enzymes for the purpose of preparing a restriction map, fragments cleaved with a first restriction enzyme were isolated by agarose gel electrophoresis for separation in accordance with the method of Tanaka et al. (T. Tanaka, B. Weisblum, J. Bacteriol., 121, 354 (1975)), then condensed by ethanol precipitation, and then cleaved with a second restriction enzyme. The cleaved fragments were subjected to agarose gel electrophoresis, and each molecular weight was calculated to prepare a restriction map, which is shown in Figure 1.

(c) Measurement of the copy numbers of pAJ 228

Brevibacterium lactofermentum AJ 12147 retaining pAJ 228 was inoculated on 5 ml of CMG liquid medium containing 50 $\mu$g/ml of trimethoprim. After culturing overnight at 30°C, 0.1 ml of the culture solution was again inoculated on 5 ml of CMG liquid medium containing 50 $\mu$g/ml of trimethoprim. After culture at 30°C at an early logarithmic growth phase, 1,000 $\mu$g/ml of ampicillin was added, and the mixture was cultured for a further 2 hours. Then cells were harvested by centrifugation and suspended in 1.5 ml of tris-EDTA-NaCl buffer containing 10 mg/ml Of lysozyme. After incubating at 37°C for 2 hours, SDS (final concentration of 4%) was added, and the cells were lysed at 65°C for 20 minutes. After it was confirmed that protoplasts had been completely lysed, extraction was performed with phenol. Then a 2-fold volume of ethanol was added to precipitate DNAs at -20°C. The precipitates were suspended in a small quantity of Tris-EDTA-NaCl buffer. After the resulting DNA solution was treated with ribonuclease (reacted with 50 $\mu$g/ml of ribonuclease I at 37°C for 60 minutes), extraction was again performed with phenol. Then a 2-fold amount of ethanol was added to precipiate DNAs at -20°C. The precipitates were suspended in a small quantity of Tris-EDTA-NaCl buffer. Thereafter the suspension was subjected to agarose gel electrophoresis. The resulting negative film was applied to a densitometer to measure the ratio of chromosomal DNAs to plasmid DNAs and to determine molecular weights of chromosomal DNAs and pAJ 228 as being $3.0 \times 10^9$ daltons and $5.0 \times 10^6$ daltons, respectively. Determination of the copy numbers by calculation revealed that 16 copies were present per chromosome. The copy numbers of pAM 330 determined in a similar manner was 15 copies.

Example 2

After Corynebacterium glutamicum (ATCC 13060) was transformed using pAJ 228 in accordance with the protoplast transformation method shown in Example 1, cells were selected by trimethoprim-resistance to obtain transformants. Further, it was confirmed by agarose gel electrophoresis that the transformants contained pAJ 228.

Example 3

Miniaturization of pAJ 228

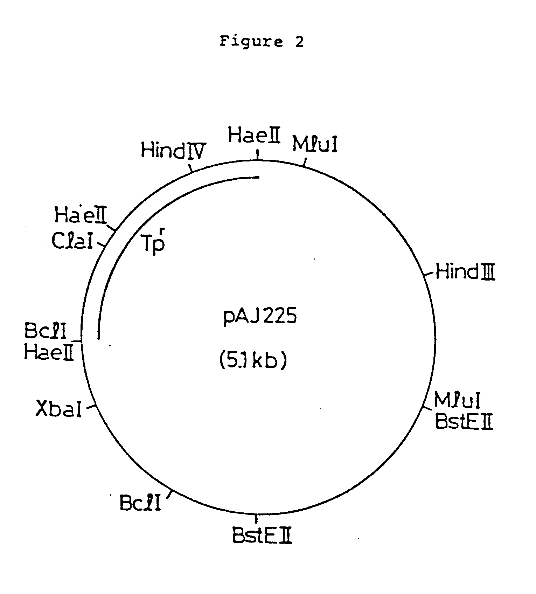

DNAs, 5 $\mu$g, of pAJ 228 prepared in Example 1 were treated with restriction endonuclease Hae II (5 units) at 30°C for 30 minutes to partially cleave them. Thereafter, the same procedure as in Example 1 was repeated to perform a ligation reaction of DNA strands with DNA ligase derived from T$_4$ phage. After completion of the reaction, heat treatment was carried out at 65°C for 10 minutes. After the addition of a 2-fold amount of ethanol, the precipitated and collected DNAs were subjected to protoplast transformation in a manner similar to Example 1, using as the recipient cell Brevibacterium lactofermentum AJ 12036 sensitive to trimethoprim. Thereafter cultivation was performed in protoplast regeneration medium containing 100 $\mu$g/ml of trimethoprim. As a result, about 100 regenerated colonies appeared, among which 10 colonies were selected, and a lysate was prepared therefrom in a manner as described in Example 1. By detection of plasmid DNAs by agarose gel electrophoresis, one plasmid obviously smaller than pAJ 228 was detected. This plasmid was named pAJ 225. The molecular weight of pAJ 225 was determined to be 5.1 Kb by an analysis similar to Example 1 using agarose gel electrophoresis. In Figure 2, a restriction map of pAJ 225 is shown. Plasmid pAJ 225 has a structure eliminating about 2.5 Kb of DNA fragments which are cleaved with restriction enzyme Hae II out of pAJ 228 and is a useful plasmid vector in which larger DNA fragments can be incorporated by miniaturization, as compared to pAJ 228.

Example 4

Introduction of Pst I cleavage site into pAJ 228

Plasmid pAJ 228 carries the cleavage sites for restriction enzymes as shown in Table 1, but as the site for one cleavage, Cla I, Hpa I, Sma I, Xba I, Xho I and Xma I alone are known. On the other hand, in plasmid vectors capable of replicating in Coryneform bacteria hitherto used, the cleavage site with restriction enzyme Pst I has been utilized as a useful site for insertion of genes (Published European Patent Application 0 093 611). Therefore, a Pst I cleavage site was introduced in pAJ 228.

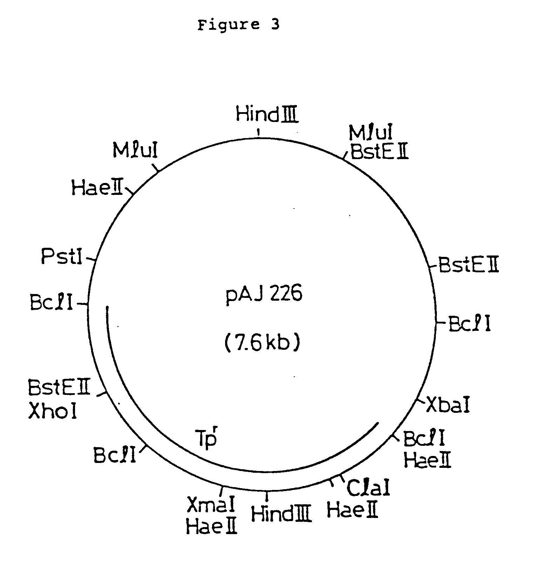

DNAs, 0.1 $\mu$g, of pAJ 228 were treated with restriction endonuclease Hpa I at 37°C for 2 hours. After completely cleaving them, heat treatment was carried out at 65°C for 10 minutes. To the reaction solution, 100 pmole of Pst I linker d (pG-C-T-G-C-A-G-C) manufactured by Takara Brewery Co., Ltd., whereby a ligation reaction of DNA strands was carried out with T$_4$ phage-derived DNA ligase at 10°C for 24 hours in the presence of ATP and dithiothreitol. After heat treatment at 65°C for 5 minutes, a 2-fold amount of ethanol was added to the reaction solution to precipitate and harvest DNAs after completion of the ligation reaction. The thus-obtained DNAs were introduced into Brevibacterium lactofermentum AJ 12036 sensitive to trimethoprim by the protoplast transformation method, in a manner similar to Example 1. Then cultivation was performed in protoplast regeneration medium containing 100 $\mu$g/ml of trimethoprim. As a result, 100 regenerated colonies appeared, among which 10 strains were selected, and a lysate was prepared by the method described in Example 1. By the detection of plasmid DNAs by agarose gel electrophoresis, plasmids having almost the same size as that of pAJ 228 were detected in any case. After these plasmids were treated with restriction enzyme Pst I or Hpa I at 37°C for 2 hours and completely cleaved, plasmid DNAs were detected by agarose gel electrophoresis, whereby a plasmid cleaved with Pst I but not with Hpa I was detected. The plasmid was named pAJ 226. A restriction map of pAJ 226 is shown in Figure 3. In pAJ 226, Pst I linker was inserted at the Hpa I cleavage site of pAJ 228 and as a result, the Hpa I cleavage site disappeared, and the Pst I cleavage site was formed.

8

Example 5

Formation of expression vector from pAJ 228

When genetic DNA is incorporated into a plasmid vector and a promoter in charge of expression of the gene is present on the incorporated genetic DNA, genetic expression is achieved by the action of the promoter. However, when no promoter is present on the incorporated genetic DNA, it is necessary to express the gene utilizing a promoter in the vector. Accordingly, vectors in which a cleavage site for insertion of genetic DNA with a restriction enzyme is positioned downstream of the promoter region possessed by the vectors are extremely useful as vectors for expression. Plasmid vectors pAJ 228, pAJ 225 and pAJ 226 shown in Examples 1, 3 and 4 had poor utility as expression vectors since the cleavage site for insertion of genetic DNAs with restriction enzyme was not always located downstream of the promoter region. Thus, in this example, there is shown a method for forming expression vectors having the cleavage site wtih restriction enzyme located downstream from the promoter region present in pAJ 228.

For detecting the promoter region in pAJ 228, it is convenient to use as a probe a DNA fragment that carries no promoter region and expresses in Coryneform bacterial cells a phenotype detectable in the case of incorporating it downstream from the promoter region. As such a DNA fragment a DNA fragment of 2.9 Kb which is derived from Brevibacterium lactofermentum AJ 11188 (FERM-P 4190) and codes for homoserine kinase (HK) was used. This DNA fragment can be cleaved out of recombinant plasmid pAJ 211 inserted with HK genes presented in Brevibacterium lactofermentum AJ 12079 (FERM-BP 578) through partial cleavage with restriction enzyme Pst I. In this DNA fragment, no promoter region was present. It was confirmed that no expression of HK genes was noted when inserted into Pst I cleavage site of pAJ 226 shown in Example 4, irrespective of the direction of the insertion.

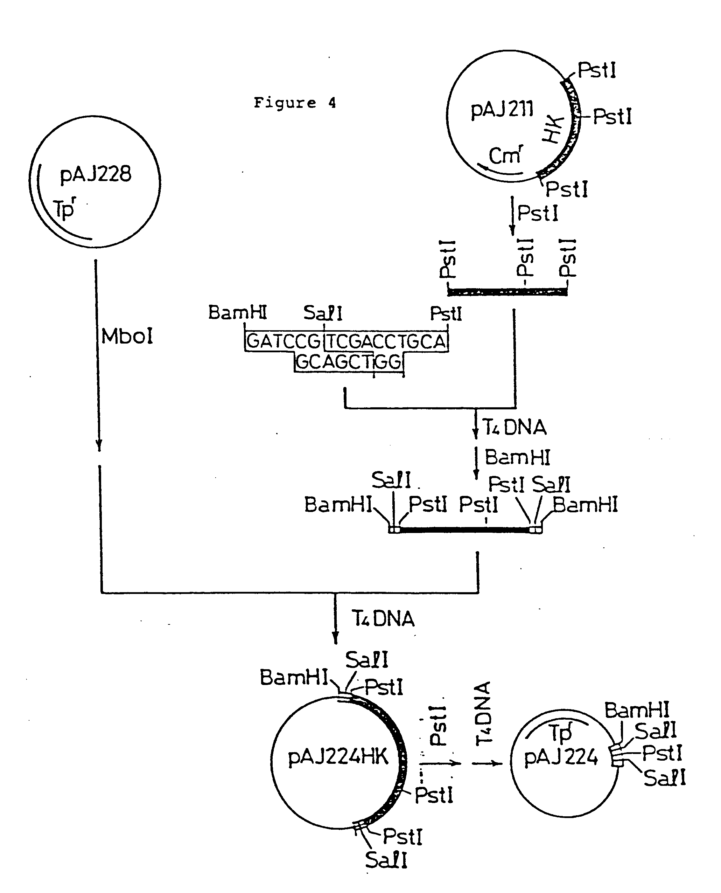

Next, a method for constructing an expression vector from pAJ 228 using this DNA fragment harboring HK genes will be stated below. An outline of the method is shown in Figure 4. At both terminals of a DNA fragment of 2.9 Kb cleaved out of pAJ 211 through partial cleavage with restriction enzyme Pst I, a synthetic oligonucleotide linker having cleavage sites of BamH I, Sal I and Pst I as shown in Figure 4 was ligated with DNA ligase derived from $T_4$ phage. Then cleavage was performed with restriction enzyme BamH I. The thus-obtained DNA mixture was subjected to agarose gel electrophoresis to separate and extract a DNA fragment of about 2.9 Kb. By ethanol precipitation, the DNA fragment was recovered. The thus-obtained DNA fragment has a structure in which the cleavage sites with Pst I, Sal I and BamH I were aligned at both terminals of DNAs containing the 2.9 Kb of HK genes (Figure 4).

On the other hand, pAJ 228 was partially cleaved with restriction enzyme Mbo I followed by heat treatment at 65$^°$C for 10 minutes. The DNA fragment containing HK genes obtained in the method described above was added to the resulting reaction solution to perform a ligation reaction of DNA strands with $T_4$ DNA ligase. After completion of the reaction, heat treatment was carried out at 65$^°$C for 5 minutes. By the addition of a 2-fold amount of ethanol, precipitated and harvested DNAs were subjected to protoplast transformation in a manner similar to Example 1, using recipient Brevibacterium lactofermentum AJ 12078 sensitive to trimethoprim and deficient for HK gene. Thereafter, cultivation was performed in protoplast regeneration medium containing 100 μg/ml of trimethoprim. As a result, about 200 regenerated colonies appeared.

The colonies were replicated in minimum medium containing 200 μg/ml of trimethoprim but containing no threonine required by recipient to obtain 4 strains resistant to trimethoprim and having lost theonine auxotrophy. From these strains, lysates were prepared in a manner similar to Example 1. By detection of plasmid DNAs by agarose gel electrophoresiss, plasmids of 14.9 Kb, 12.2 Kb, 9.6 Kb and 6.5 Kb were detected. Of these, the plasmid having the smallest molecular weight was named pAJ 224 HK. AJ 12076 introduced with pAJ 244 HK showed very high activity of HK.

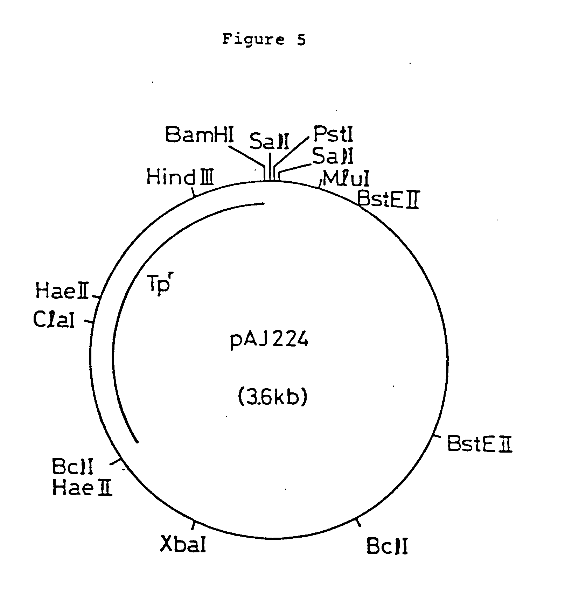

Next, pAJ 224 HK was completely cleaved with restriction enzyme Pst I. Thereafter, a 3.6 Kb fragment which was believed to be miniaturized vector pAJ 228 was recovered from agarose electrophoresis gel. The fragment was subjected, in a manner similar to Example 1, to a ligation reaction of DNA strands with $T_4$ DNA ligase and protoplast transformation using as recipient Brevibacterium lactofermentum AJ 12036 sensitive to trimethoprim. After culturing in protoplast regeneration medium containing 100 μg/ml of trimethoprim, about 100 regenerted colonies were thus obtained, from which 10 strains were selected, and lysates were prepared therefrom in a manner similar to Example 1. By detection of plasmid DNAs by agarose gel electrophoresis, plasmid DNA of 2.9 Kb which was believed to have DNA of 2.9 Kb containing HK genes removed from pAJ 224 HK was detected. The plasmid was named pAJ 224, and a strain harboring this pAJ 224 was named Brevibacterium lactofermentum AJ 12196 (FERM-P 8015 FERM BP-787). A restriction map of pAJ 224 is shown in Figure 5.

Plasmid pAJ 224 has a function as expression vector. It can be confirmed, as stated above, from the fact that when a DNA fragment of HK genes carrying no promoter region is inserted at the cleavage site with restriction enzyme Pst I, threonine auxotrophy of HK-deficient strain disappears. Further for the purpose of examining the intensity of its expression activity, the following experiment was carried out.

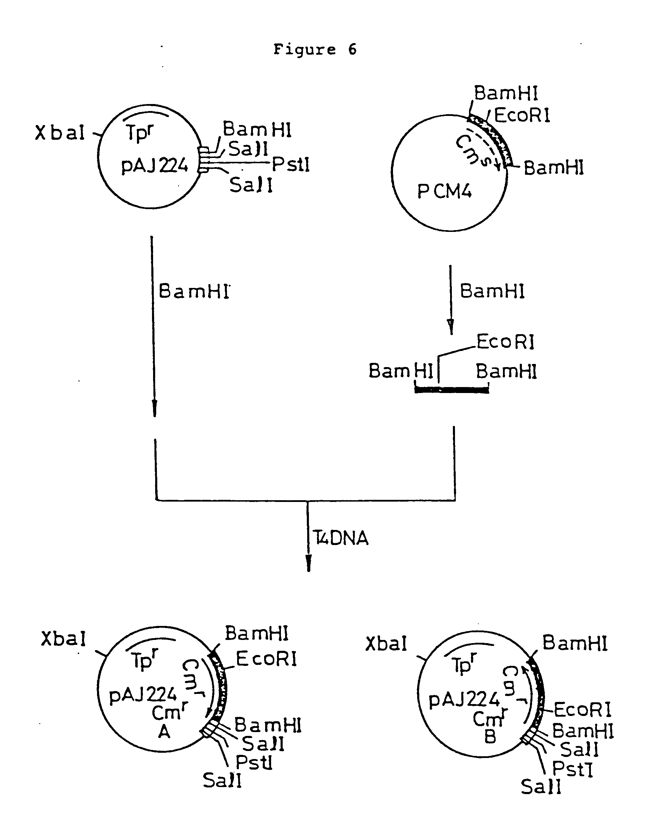

As an index for the intensity of the expression activity, resistance to chloramphenicol, which expresses in Coryneform bacterial cells and is easily measurable, has been used. As shown in Figure 6, a structural genetic region excluding a promoter region of chloramphenicol-resistant gene was cleaved out of DNAs of plasmid pCM 4 (cf. Gene, 20, 305-306 (1982)) using restriction enzyme BamH I and mixed with DNAs of pAJ 224 completely cleaved with BamH I similarly, and the mixture was subjected to a ligation reaction of DNA strands with $T_4$ DNA ligase. In a manner similar to Example 1, thermal denaturation and ethanol precipitation were performed. The resulting DNAs were subjected to protoplast transformation using as recipient Brevibacterium lactofermentum AJ 12036 sensitive to chloramphenicol. Thereafter, cultivation was carried out in protoplast regeneration medium containing 2 $\mu$g/ml of chloramphenicol to obtain renegerated colonies resistant to chloramphenicol. From these chloramphenicol-resistant strains, 10 strains were selected, and lysates were prepared therefrom in a manner similar to Example 1. By detection of plasmid DNAs through agarose gel electrophoresis all of them were plasmids of 4.4 Kb having inserted in pAJ 224 DNA fragment containing a chloramphenicol-resistant, structural genetic region derived from pCM 4 available from Pharmacia, No. 27492901. The plasmid was named pAJ 224 Cm$^r$.

Next, Brevibacterium lactofermentum AJ 12036 harboring pAJ 224 Cm$^r$ was compared with other strains in resistance to chloramphenicol. The results are shown in Table 2. As a representative of chloramphenicol-resistant strains, plasmid pAJ 1844 (Published Unexamined European Patent Application 0 093 611) having the entire region of chloramphenicol-resistant gene was used as a control. As is clear from Table 2, it was confirmed that pAJ 224 was highly useful as expression vector since the strain harboring pAJ 224 Cm$^r$ showed chloramphenicol-resistance equivalent to the strain harboring pAJ 1844. Further, analysis with respect to the direction for the insertion of the structural genetic region of chloramphenicol-resistant gene in pAJ 224 Cm$^r$ was performed by cleavage patterns with restriction enzymes Xba I and EcoR I. The results revealed that the resulting pAJ 224 Cm$^r$ included one inserted with the structural genetic region into trimethoprim-resistant gene side as the upper flow (5' site) (pAJ 224 Cm$^r$-A) and one inserted into the opposite direction (pAJ 224 Cr$^r$-B). This indictes that in pAJ 224, promoter regions are present on both sides with the cleavage sites with restriction enzymes BamH I, Sal I Pst I between and also indicates that the gene inserted into the cleavage sites has a possibility of expression, irrespective of the direction inserted.

Example 6

Construction of pAJ 223

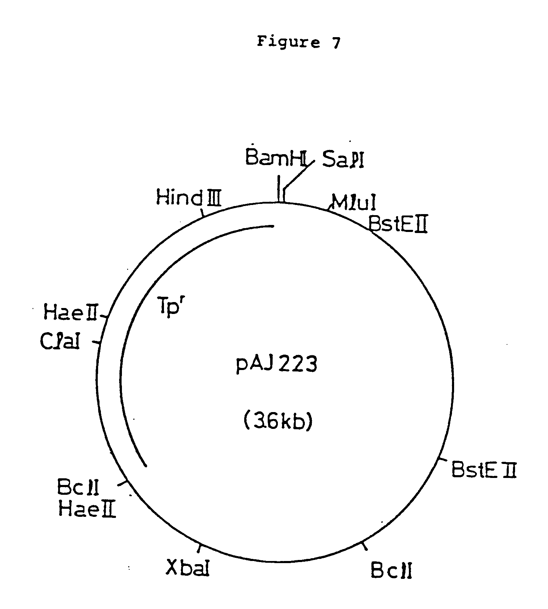

After pAJ 224 HK produced as in Example 5 was completely cleaved by restriction enzyme Sal I, a ligation reaction of DNA strands was carried out with $T_4$ DNA ligase. In a manner similar to Example 1, thermal denaturation and ethanol precipitation were carried out. The thus-obtained DNAs were subjected to protoplast transformation using Brevibacterium lactofermentum AJ 12036 sensitive to trimethoprim and cultured in protoplast regeneration medium. Regenerated colonies which appeared were replicated in minimum medium containing trimethoprim, and trimethoprim-resistant strains were selected. From these resistant strains, 20 strains were selected, and lysates were pepared in a manner similar to Example 1. By detection of plasmid DNAs by agarose gel electrophoresis, plasmid DNA of 3.6 Kb almost the same as pAJ 224 shown in Example 5 was detected. This plasmid was named pAJ 223. A restriction map of pAJ 223 is shown in Figure 7. Plasmid pAJ 223 has a structure from which the cleavage site with restriction enzyme Pst I is removed from pAJ 224.

## Table 2

| Host Bacterium Plasmid | Concentration of Chloramphenicol added (μg/ml) | | | | |
|---|---|---|---|---|---|
| | 0 | 10 | 20 | 40 | 80 |
| AJ 12036 none | + | − | − | − | − |
| AJ 12036 pAJ 224 | + | − | − | − | − |
| AJ 12036 pAJ 224 Cm$^r$ | + | + | + | + | + |
| AJ 12036 pAJ 1844 | + | + | + | + | ± |

+: grows

±: grows slightly

−: does not grow

Each bacterium was inoculated on agar plate of complete medium containing chloramphenicol and cultured at 30°C for 24 hours.

## Claims

1. A recombinant plasmid capable of replicating in a Coryneform bacterial cell, which comprises:
   a first segment comprising a replication site capable of causing replication of said plasmid in a Coryneform bacterial cell and
   a second segment comprising a Brevibacterium lactofermentum gene capable of conferring trimethoprim resistance obtainable from any of the plasmids pAJ 228 deposited in strain AJ 12 147 (FERM-P 7673 = FERM-BP 786) or pAJ 224 deposited in strain AJ 12 196 (FERM-P 8015 = FERM-BP 787).

2. The recombinant plasmid of Claim 1, wherein expression of said second segment in a Coryneform bacterium results in resistance to 100 μg/ml trimethoprim.

3. The recombinant plasmid of Claim 2, wherein said resistance is 200 μg/ml.

4. The recombinant plasmid of Claim 1, wherein said first segment is substantially identical to a plasmid segment in a plasmid having a Coryneform bacterium as its normal host.

5. The recombinant plasmid of Claim 4, wherein said first segment is identical to said plasmid segment.

6. The recombinant plasmid of Claim 1, wherein said plasmid is a member selected from the group consisting of
   - pAJ 224 (FERM-BP 787),
   - pAJ 228 (FERM-BP 786),
   - pAJ 225, obtainable by partial Hae II-cleavage of pAJ 228, ligation and selection for the 5.1kb product,
   - pAJ 226, obtainable by cleaving pAJ 228 with Hpa I, ligating it to a Pst I-linker, recircularizing it and selecting for a 7.6kb plasmid carrying a Pst I-site but lacking the Hpa I-site of pAJ 228,

- pAJ 224 HK, obtainable by partial Pst I-cleavage of pAJ 211 (FERM-BP 578) to produce a 2.9kb HK gene-carrying fragment, ligating this fragment to a linker, again ligating this construct to partially Mbo I-digested pAJ 228, and finally selecting for the 6.5kb-sized plasmid,
- pAJ 223, obtainable by Sal I-digestion of pAJ 224 HK, ligation and selection for the 3.6kb trimethoprim resistant plasmid, and
- pAJ 224 Cm$^r$, obtainable by BamH I-excision of the chloramphenicol resistance gene out of pCM4, ligation of it to BamH I-cleaved pAJ 224 and selection for the chloramphenicol resistant construct of 4.4kb.

7. Recombinant plasmids pAJ 228, deposited as FERM BP 786, and pAJ 224, deposited as FERM BP 787.

8. A method of producing a recombinant plasmid capable of transforming a Coryneform bacterium, which comprises:

ligating (1) a DNA segment containing a replication site capable of causing replication of a plasmid in a Coryneform bacterial cell with (2) a DNA segment comprising a Brevibacterium lactofermentum gene capable of conferring trimethoprim resistance obtainable from any of the plasmids pAJ 228 deposited in strain AJ 12 147 (FERM-P 7673 = FERM-BP 786) or pAJ 224 deposited in strain AJ 12 196 (FERM-P 8015 = FERM-BP 787).

9. A transformed Coryneform bacterium containing the recombinant plasmid of any of the claims 1 to 7.

**Revendications**

1. Plasmide recombiné capable de réplication dans une cellule de bactérie Corynéforme qui comprend :

un premier segment comprenant un site de réplication capable de provoquer la réplication dudit plasmide dans une cellule de bactérie Corynéforme et

un second segment comprenant un gène de Brevibacterium lactofermentum capable de conférer une résistance au triméthoprime pouvant être obtenu à partir de l'un quelconque des plasmides pAJ 228 déposé dans la souche AJ 12 147 (FERM-P 7673 = FERM-BP 786) ou pAJ 224 déposé dans la souche AJ 12 196 (FERM-P 8015 = FERM-BP 787).

2. Plasmide recombiné selon la revendication 1, dans lequel l'expression dudit second segment dans une bactérie Corynéforme provoque une résistance à 100 $\mu$g/ml de triméthoprime.

3. Plasmide recombiné selon la revendication 2, dans lequel ladite résistance est à 200 $\mu$g/ml.

4. Plasmide recombiné selon la revendication 2, dans lequel ledit premier segment est sensiblement identique à un segment de plasmide dans un plasmide ayant une bactérie Corynéforme comme hôte normal.

5. Plasmide recombiné selon la revendication 4, dans lequel ledit premier segment est identique audit segment de plasmide.

6. Plasmide recombiné selon la revendication 1, dans lequel ledit plasmide fait partie du groupe constitué par
- pAJ 224 (FERM-BP 787),
- pAJ 228 (FERM-BP 786),
- pAJ 225, pouvant être obtenu par clivage partiel par Hae II de pAJ 228, ligature et sélection du produit de 5,1 kb,
- pAJ 226, pouvant être obtenu par clivage de pAJ 228 avec Hpa I, ligature à un linker Pst I, recircularisation et sélection d'un plasmide de 7,6 kb portant un site Pst I, mais dépourvu du site Hpa I de pAJ 228,
- pAJ 224 HK, pouvant être obtenu par clivage partiel par Pst I de pAJ 211 (FERM-BP 578) pour produire un fragment portant le gène HK de 2,9 kb, ligature de ce fragment à un linker, ligature à nouveau de ce produit d'assemblage à pAJ 228 ayant subi une digestion partielle par Mbo I et finalement sélection du plasmide ayant une taille de 6,5 kb,
- pAJ 223, pouvant être obtenu par digestion par Sal I de pAJ 224 HK, ligature et sélection du plasmide de 3,6 kb résistant au triméthoprime et

- pAJ 224 Cm$^r$, pouvant être obtenu par excision par BamH I du gêne de résistance au chloramphénicol de pCM4, ligature de celui-ci à pAJ 224 clivé avec BamH I et sélection du produit d'assemblage de 4,4 kb résistant au chloramphénicol.

7. Plasmides recombinés pAJ 228 déposé comme FERM-BP 786 et pAJ 224 déposé comme FERM-BP 787.

8. Procédé pour produire un plasmide recombiné capable de transformer une bactérie Corynéforme, qui comprend :

la ligature de (1) un segment d'ADN contenant un site de réplication capable de provoquer la réplication d'un plasmide dans une cellule de bactérie Corynéforme avec (2) un segment d'ADN comprenant un gène de Brevibacterium lactofermentum capable de conférer une résistance au triméthoprime pouvant être obtenu à partir de l'un quelconque des plasmides pAJ 228 déposé dans la souche AJ 12 147 (FERM-P 7673 = FERM-BP 786) ou pAJ 224 déposé dans la souche AJ 12 196 (FERM-P 8015 = FERM-BP 787).

9. Bactérie Corynéforme transformée contenant le plasmide recombiné de l'une quelconque des revendications 1 à 7.

## Patentansprüche

1. Rekombinantes Plasmid mit der Fähigkeit zur Replikation in einer coryneformen Bakterienzelle, das enthält :

ein erstes Segment, das eine Replikationsstelle enthält mit der Fähigkeit, die Replikation des Plasmids in einer coryneformen Bakterienzelle zu bewirken und

ein zweites Segment, das ein Brevibacterium lactofermentum-Gen enthält, das befähigt ist, Trimethoprim-Resistenz zu verleihen, und das von einem der Plasmide pAJ 228, hinterlegt im Stamm AJ 12 147 (FERM-P 7673 = FERM-BP 786) oder pAJ 224, hinterlegt im Stamm AJ 12 196 (FERM-P 8015 = FERM-BP 787), erhältlich ist.

2. Rekombinantes Plasmid nach Anspruch 1, wobei die Expression des zweiten Segments in einem coryneformen Bakterium eine Resistenz von 100 $\mu$g/ml Trimethoprim bewirkt.

3. Rekombinantes Plasmid nach Anspruch 2, wobei die Resistenz 200 $\mu$g/ml ist.

4. Rekombinantes Plasmid nach Anspruch 1, wobei das erste Segment im wesentlichen identisch ist mit einem Plasmidsegment in einem Plasmid, das ein cornyeformes Bakterium als normalen Wirt hat.

5. Rekombinantes Plasmid nach Anspruch 4, wobei das erste Segment identisch mit diesem Plasmidsegment ist.

6. Rekombinantes Plasmid nach Anspruch 1, wobei das Plasmid ausgewählt ist unter
   - pAJ 224 (FERM-BP 787),
   - pAJ 228 (FERM-BP 786),
   - pAJ 225, erhältlich durch teilweise Hae II-Spaltung von pAJ 228, Verknüpfen und Selektion des 5,1kb-Produkts,
   - pAJ 226, erhältlich durch Spaltung von pAJ 228 mit Hpa I, dessen Verknüpfung mit einem Pst I-Verbindungsstück, dessen erneuter Zirkularisierung und Selektion eines 7,6 kb Plasmids, das eine Pst I-Stelle, aber keine Hpa I-Stelle von pAJ 228 enthält,
   - pAJ 224 HK, erhältlich durch teilweise Pst I-Spaltung von pAJ 211 (FERM-BP 578), um ein Fragment herzustellen, das ein 2,9 kb HK-Gen enthält, Verknüpfen dieses Fragments mit einem Verbindungsstück, Wiederverknüpfen dieses Konstrukts mit einem teilweise Mbo I-verdauten pAJ 228, und schließlich Selektion des 6,5kb großen Plasmids,
   - pAJ 223, erhältlich durch Sal I-Verdauung von pAJ 224 HK, Verknüpfen und Selektion des 3,6kb Trimethoprimresistenten Plasmids, und
   - pAJ 224 Cm$^r$, erhältlich durch BamH I-Exzision des Chloramphenicol-Resistenz-Gens aus pCM4, dessen Verknüpfung mit BamH I-gespaltenen pAJ 224 und Selektion des Chloramphenicol-resistenten Konstrukts von 4,4kb.

7. Rekombinante Plasmide pAJ 228, hinterlegt als FERM-BP 786, und pAJ 224, hinterlegt als FERM-BP 787.

8. Verfahren zur Herstellung eines rekombinanten Plasmids mit der Fähigkeit zur Transformation eines coryneformen Bakteriums, umfassend :

Verknüpfen (1) eines DNA-Segments, das eine Replikationsstelle enthält, mit der Fähigkeit, die Replikation eines Plasmids in einer coryneformen Bakterienzelle zu bewirken, mit (2) einem DNA-Segment, das ein Brevibacterium lactofermentum-Gen enthält, mit der Fähigkeit, Trimethoprim-Resistenz zu übertragen, das erhältlich ist aus einem der Plasmide pAJ 228, hinterlegt im Stamm AJ 12 147 (FERM-P 7673 = FERM-BP 786) oder pAJ 224, hinterlegt im Stamm AJ 12 196 (FERM-P 8015 = FERM-BP 787).

9. Transformiertes coryneformes Bakterium, das ein rekombinantes Plasmid nach einem der Ansprüche 1 bis 7 enthält.

Figure 1

Figure 2

Figure 3

Figure 4

Figure 5

Figure 6

Figure 7